(19) Europäisches Patentamt
European Patent Office
Office européen des brevets

(11) **EP 2 153 793 A2**

(12) **EUROPÄISCHE PATENTANMELDUNG**

(43) Veröffentlichungstag:
**17.02.2010 Patentblatt 2010/07**

(51) Int Cl.:
***A61B 19/00*** *(2006.01)*

(21) Anmeldenummer: **09010328.4**

(22) Anmeldetag: **11.08.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **13.08.2008 DE 102008038911**

(71) Anmelder: **Technische Universität Darmstadt 64289 Darmstadt (DE)**

(72) Erfinder: **Röse, Andreas 64289 Frankfurt (DE)**

(74) Vertreter: **Katscher Habermann Patentanwälte Dolivostrasse 15A 64293 Darmstadt (DE)**

(54) **Manipulationsvorrichtung für ein chirurgisches Instrument und chirurgisches Instrument**

(57)     Eine Manipulationsvorrichtung (1) für ein chirurgisches Instrument mit einer Befestigungseinrichtung zur Befestigung der Manipulationsvorrichtung (1) an einem Instrumentenschaft, an welchem mehrere Linearaktoren (2) festlegbar sind, mit einer Werkzeugplattform (8) zur Aufnahme von mindestens einem chirurgischen Werkzeug und mit mehreren passiven Stab-Gelenk-Einheiten (5,9,10,11), deren eines Ende (6) jeweils mit einem zugeordneten Linearaktor (2) verbindbar ist und mittels derer die Werkzeugplattform (8) mit den Linearaktoren (2) in Wirkverbindung bringbar ist, weist mindestens zwei durchgehende Stab-Gelenk-Einheiten (10,11) auf, die jeweils mit der werkzeugplattform (8) verbunden sind und mindestens eine verzweigende Stab-Gelenk-Einheit (10,11), die an einem Verzweigungsabschnitt (12) beabstandet zu der Werkzeugplattform (8) mit einer zugeordneten durchgehenden Stab-Gelenk-Einheit (5) verbunden ist.

Fig. 4

**Beschreibung**

Manipulationsvorrichtung für ein chirurgisches Instrument und chirurgisches Instrument

Die Erfindung betrifft eine Manipulationsvorrichtung für ein chirurgisches Instrument mit einer Befestigungseinrichtung zur Befestigung der Manipulationsvorrichtung an einem Instrumentenschaft, an welchem mehrere Linearaktoren festlegbar sind, mit einer Werkzeugplattform zur Aufnahme von mindestens einem chirurgischen Werkzeug und mit mehreren passiven Stab-Glenk-Einheiten, deren eines Ende jeweils mit einem zugeordneten Linearaktor verbindbar ist und mittels derer die werkzeugplattform mit den Linearaktoren in Wirkverbindung bringbar ist.

Aus der Praxis sind verschiedene Manipulationsvorrichtungen für ein chirurgisches Instrument bekannt. Bei minimalinvasiven chirurgischen Eingriffen und insbesondere derartigen Eingriffen in dem Bauchraum sind lange, stabförmige Instrumente vorteilhaft, die durch kleine Zugänge, sogenannte Trokare, in den Körper eingeführt werden können.

Ist die an einem Ende des stabförmigen Instruments angeordnete Werkzeugplattform starr mit dem stabförmigen Instrument verbunden, so können die an der Werkzeugplattform angeordneten chirurgischen Instrumente nur mit einer vorgegebenen Vorzugsrichtung in einem kegelförmigen Arbeitsraum eingesetzt werden.

Aus der Praxis sind beispielsweise Endoskope oder anderer chirurgische Instrumente bekannt, die einen stabförmigen Instrumentenschaft und eine damit verbundene Manipulationsvorrichtung aufweisen, an welcher die werkzeugplattform befestigt ist. Die Manipulationsvorrichtung erlaubt eine beispielsweise über Seilzüge (beispielsweise EP 1 224 918) oder elektromotorisch angetriebene Verlagerung der Werkzeugplattform relativ zu dem stabförmigen Instrumentenschaft. Eine weitere Ausgestaltung derartiger Manipulationsvorrichtungen ist beispielsweise aus US 2005096694 A bekannt.

Einfache Manipulationsvorrichtungen ermöglichen beispielsweise ein Verschwenken der Werkzeugplattform um eine Drehachse. Eine aufwendigere Konstruktion einer Manipulationsvorrichtung mit zwei Freiheitsgraden der Bewegung ist beispielsweise in US 6309403 B beschrieben.

Es sind auch komplexe Manipulationsvorrichtungen bekannt, bei denen die Werkzeugplattform über mehrere gelenkig miteinander verbundene Glieder mit dem Instrumentenschaft verbunden ist, so dass im Inneren des Körpers die Werkzeugplattform innerhalb eines großen Arbeitsraumes verlagert und gezielt zum Einsatz gebracht werden kann, obwohl das chirurgische Instrument durch einen vergleichsweise kleinen Trokar in den Körper eindringt.

Es sind weiterhin verschiedene Manipulationsvorrichtungen bekannt, die jeweils mehrere Antriebs- oder Führungsketten aufweisen, die jeweils aus gelenkig miteinander verbundenen Gliedern gebildet werden. Die einzelnen Gelenke können dabei entweder aktiv angetriebene Gelenke oder aber passive, nicht angetriebene Gelenke sein. Ein Ausführungsbeispiel mit aktiven Gelenken wird beispielsweise in US 2001018591 A beschrieben. Eine Aneinanderreihung mehrerer gelenkig miteinander verbundener Glieder kann dabei im Hinblick auf deren Funktion als Antriebs- oder Führungskette bezeichnet werden. Im Folgenden werden Aneinanderreihungen einer beliebigen Anzahl von Gliedern und Gelenken als Stab-Gelenk-Einheit bezeichnet, wobei durch diese Bezeichnung keine Einschränkung der jeweiligen Formgebung auf stabförmige Glieder erfolgen soll.

Als passive Stab-Gelenk-Einheit wird eine Kombination mehrerer Glieder mit mindestens einem Gelenk bezeichnet, wobei das mindestens eine Gelenk, bzw. jedes Gelenk in der Stab-Gelenk-Einheit ein passives, also ein nicht angetriebenes Gelenk ist.

Die aus der Praxis bekannten Manipulationsvorrichtungen sind oftmals sehr aufwendig und kostenintensiv in der Herstellung. Werden überwiegende oder ausschließlich passive Gelenke verwendet, so sind bei einer bekannten Vorrichtung viele passive Stab-Gelenk-Einheiten mit einer oftmals komplexen Formgebung und Anordnung relativ zueinander erforderlich, um dennoch lediglich eingeschränkte Arbeitsräume für das an der Werkzeugplattform befestigte chirurgische Instrument zu ermöglichen. In den meisten Fällen können die bekannten Manipulationsvorrichtungen seitlich angreifende Kräfte nur ungenügend aufnehmen, beziehungsweise diesen Stand halten, so dass dadurch der praktische Nutzen dieser Manipulationsvorrichtungen trotz des gegebenenfalls großen erreichbaren Arbeitsraums eingeschränkt ist. Insbesondere große Manipulationsvorrichtungen, wie sie beispielsweise bei Industrierobotern bekannt sind, weisen viele oder ausschließlich aktive Gelenke auf.

Es sind auch chirurgische Instrumente bekannt, bei denen die Stab-Gelenk-Einheiten der Manipulationsvorrichtung an verschiedenen Stellen mit zugeordneten aktiven Stab-Gelenk-Einheiten oder mit zugeordneten Aktuatoren in wirkverbindung stehen. Derartige chirurgische Instrumente weisen üblicherweise keinen kompakten Instrumentenschaft auf, der beispielsweise wie bei Endoskopen oder Gastroskopen zumindest teilweise im Rahmen eines minimalinvasiven Eingriffs in den Körper eingeführt werden kann. Auf Grund der oftmals an verschiedenen Stellen des chirurgischen Instruments beabstandet angeordneten Stab-Gelenk-Einheiten und des für diese Anordnung notwendigen Raumbedarfs kann die Manipulationsvorrichtung nur teilweise in den Körper eingeführt werden. Derartige chirurgische Instrumente ermöglichen regelmäßig nur einen kleinen Arbeitsraum des in den Körper eindringenden Teils der Manipulationsvorrichtung, während der überwiegende Anteil des chirurgischen Instruments außerhalb des Körpers verbleiben muss. Der Raumbedarf dieser Instrumente ist oftmals ganz erheblich größer als der tatsächlich zur Ver-

Aufgabe der vorliegenden Erfindung ist es demzufolge, eine Manipulationsvorrichtung der eingangs genannten Gattung so auszugestalten, dass die Werkzeugplattform innerhalb eines möglichst großen Arbeitsraumes verlagert werden kann und gleichzeitig eine möglichst große Steifigkeit der Manipulationsvorrichtung ermöglicht wird, um seitlich angreifende Kräfte besser aufnehmen zu können, ohne dass die auftretenden Kräfte zu einer unkontrollierbaren Verlagerung der Werkzeugplattform führen.

Diese Aufgabe wird erfindungsgemäß dadurch gelöst, dass mindestens zwei durchgehende Stab-Gelenk-Einheiten jeweils mit der Werkzeugplattform verbunden sind und dass mindestens eine verzweigende Stab-Gelenk-Einheit an einem Verzweigungsabschnitt beabstandet zu der Werkzeugplattform mit einer zugeordneten durchgehenden Stab-Gelenk-Einheit verbunden ist.

Untersuchungen haben ergeben, dass sich bei Verwendung von mindestens einer verzweigenden Stab-Gelenk-Einheit seitlich angreifende Kräfte wesentlich besser aufnehmen lassen, insbesondere wenn diese verzweigende Stab-Gelenk-Einheit dann zumindest abschnittsweise in einem Winkel zu den durchgehenden Stab-Gelenk-Einheiten verläuft und an dem Verzweigungsabschnitt schräg, beziehungsweise rechtwinklig abstehend mit der zugeordneten durchgehenden Stab-Gelenk-Einheit verbunden ist. Die Handhabung und präzise Steuerung, beziehungsweise die räumliche Führung der Manipulationsvorrichtung wird dadurch wesentlich erleichtert.

Weiterhin lassen sich derartige kinematische Strukturen mit verzweigenden Stab-Gelenk-Einheiten, beziehungsweise Antriebsketten in weitgehend abgeschlossene Unterstrukturen aufteilen, wodurch deren optimierung und die für eine geeignete Ansteuerung der Linearaktoren erforderlichen Berechnungen vereinfacht werden.

Die Befestigungseinrichtung kann eine einteilig ausgeführte Befestigungseinrichtung für eine gleichzeitige Festlegung aller Stab-Gelenk-Einheiten an dem Instrumentenschaft sein. Es ist jedoch in den meisten Fällen denkbar und oftmals vorteilhaft, wenn jede Stab-Gelenk-Einheit beispielsweise mittels eines rastenden Kopplungselements oder mittels einer Schnappverbindung unmittelbar an dem zugeordneten Linearaktor festgelegt wird, so dass die Befestigungseinrichtung aus mehreren Befestigungselementen besteht. Die Befestigungseinrichtung kann weiterhin eine einstückig oder mehrteilig ausgestaltete Zwangsführung, bzw. Linearführung für die einzelnen Stab-Gelenk-Einheiten aufweisen.

Vorzugsweise ist vorgesehen, dass zwischen dem Verzweigungsabschnitt der mit einer verzweigenden Stab-Gelenk-Einheit verbundenen durchgehenden Stab-Gelenk-Einheit und der Werkzeugplattform mindestens ein Gelenk angeordnet ist. Dieses Gelenk kann entweder einen Gelenkfreiheitsgrad oder aber mehrere Gelenkfreiheitsgrade aufweisen. Die Anordnung eines Gelenks mit einem rotatorischen Freiheitsgrad zwischen dem Verzweigungsabschnitt und der werkzeugplattform dürfte für viele Anforderungen ausreichend und für eine einfache Herstellung und Ansteuerung der Manipulationsvorrichtung vorteilhaft sein.

Gemäß einer besonders vorteilhaften Ausgestaltung des Erfindungsgedankens ist vorgesehen, dass die Manipulationsvorrichtung zwei verzweigende Stab-Gelenk-Einheiten aufweist. Aus Symmetriegründen sind die zwei verzweigenden Stab-Gelenk-Einheiten vorzugsweise an demselben Verzweigungsabschnitt mit der derselben durchgehenden Stab-Gelenk-Einheit verbunden. Auf diese Weise kann mit einfachen konstruktiven Mitteln sicher gestellt werden, dass sich die Manipulationsvorrichtung in Richtung der beiden üblicherweise symmetrisch angeordneten verzweigenden Stab-Gelenk-Einheiten gleichermaßen auslenken und verlagern lässt.

Ein für viele praktische Anwendungen ausreichend großer Arbeitsraum der Werkzeugplattform lässt sich mit einfachen konstruktiven Mitteln dadurch erschließen, beziehungsweise zugänglich machen, dass die Manipulationsvorrichtung zwei verzweigende Stab-Gelenk-Einheiten und zwei durchgehende Stab-Gelenk-Einheiten aufweist. Die insgesamt vier Stab-Gelenk-Einheiten können mit insgesamt vier Linearaktoren verbunden werden und eine Manipulationsvorrichtung mit vier Bewegungsfreiheitsgraden ermöglichen. Vorzugsweise sind die beiden verzweigenden Stab-Gelenk-Einheiten mit derselben durchgehenden Stab-Gelenk-Einheit verbunden.

Dabei werden zweckmäßiger weise neben der durch die Linearaktoren bereits vorgegebenen Linearbewegung der Werkzeugplattform drei rotatorische Freiheitsgrade vorgesehen, da diese den höchsten Nutzen bei einer chirurgischen Verwendung der Manipulationsvorrichtung aufweisen.

In Abhängigkeit von einer vorgegebenen Anzahl von Bewegungsfreiheitsgraden der Manipulationsvorrichtung sowie der Anzahl der verwendeten Stab-Gelenk-Einheiten und der vorgesehenen Verzweigungen lässt sich die Anzahl der auf die einzelnen Gelenke entfallenden Gelenkfreiheitsgrade ermitteln, die eine mechanisch ausreichend bestimmte, jedoch nicht mechanisch überbestimmte Manipulationsvorrichtung aufweisen sollte. Die auf die einzelnen Stab-Gelenk-Einheiten entfallenden Gelenkfreiheitsgrade können dabei weitgehend beliebig auf die Stab-Gelenk-Einheiten, bzw. die jeweils in der betreffenden Stab-Gelenk-Einheit angeordneten Gelenke verteilt werden, wobei natürlich grundsätzlich beachtet werden muss, dass die Manipulationsvorrichtung ausreichend mechanisch bestimmt und nicht überbestimmt ist und eine zuverlässige Ansteuerung der Manipulationsvorrichtung innerhalb des Arbeitsraums gewährleistet werden sollte.

Es hat sich hinsichtlich der Herstellung und der Zuverlässigkeit der Betätigung der Manipulationsvorrichtung als vorteilhaft herausgestellt, wenn die Stab-Gelenk-Einheiten ausschließlich Rotationsgelenke mit einem rotatorischen Gelenkfreiheitsgrad aufweisen. Geeignete Rotationsgelenke können auch mit äußerst geringen Abmessungen in miniaturisierter Form beispielsweise als Schwenkgelenke oder Knickgelenke kostengünstig und schnell in großer Anzahl hergestellt werden.

In besonders vorteilhafter weise können Stab-Gelenk-Einheiten mit derartigen Rotationsgelenken auch einstückig ausgestaltet und hergestellt sein. So kann beispielsweise ein Schwenkgelenk dadurch hergestellt werden, dass ein stabförmiges Gebilde in einem Teilabschnitt an gegenüberliegenden, einander zugeordneten Seiten mit geeigneten Ausnehmungen versehen wird. Die zu beiden Seiten der Ausnehmungen verbleibenden Teilbereiche des stabförmigen Gebildes lassen sich dann senkrecht zu einer gedachten Verbindungslinie der beiden Ausnehmungen relativ zueinander verschwenken, wobei in dem durch die Ausnehmungen geschwächten Bereich lokal beschränkt eine Verformung stattfindet. Derartige rotatorische Gelenke sind in besonders vorteilhafter Weise für eine monolithische Herstellung beispielsweise gemäß den vorangehenden Ausführungen geeignet. Die einzelnen Stab-Gelenk-Einheiten oder die gesamte Manipulationsvorrichtung bestehend aus mehreren Stab-Gelenk-Einheiten können auf diese Weise einstückig hergestellt werden, indem an einer Werkzeugplattform ausgebildete, durchgehende Führungsketten mit einer im Wesentlichen gleichbleibenden, beispielsweise rechteckigen oder kreisrunden Querschnittsfläche durch geeignet angeordnete und ausgestaltete Schwächungsbereiche, die Schwenkgelenke bilden, in passive Stab-Gelenk-Einheiten umgewandelt werden. Die Manipulationsvorrichtung kann kostengünstig aus Kunststoff hergestellt und dabei beispielsweise mit geeigneten Spritzgusstechniken oder aus einem Flächengebilde vorgeformt und gegebenenfalls anschließend nachbearbeitet werden. Die Manipulationsvorrichtung kann als Einweg-Artikel in einfacher Weise lösbar mit dem Instrumentenschaft und den zugeordneten Linearaktoren verbindbar sein und nach Gebrauch abgelöst und entsorgt werden, so dass eine aufwendige und kostenintensive Reinigung der Manipulationsvorrichtung entfällt und eine Kontamination des behandelten Körpers durch mehrfache Benutzung der Manipulationsvorrichtung ausgeschlossen werden kann.

Es ist jedoch ebenfalls denkbar und für einige Anwendungsbereiche vorteilhaft, wenn mindestens ein Gelenk mehrere Freiheitsgrade aufweist und beispielsweise als Kardangelenk oder als Kugelgelenk ausgebildet ist.

Es hat sich gezeigt, dass ein großer Arbeitsraum dadurch erreicht werden kann, dass die jeweils benachbarten Rotationsgelenke relativ zueinander verdrehte Gelenkachsen aufweisen. Als besonders vorteilhaft hat sich herausgestellt, dass die Gelenkachsen mehrerer benachbarter Rotationsgelenke innerhalb einer Stab-Gelenk-Einheit einen Winkel von etwa 90 Grad relativ zueinander aufweisen.

Um trotz einer Befestigung der Manipulationsvorrichtung an einem Instrumentenschaft mit einer kleinen Grundfläche, bzw. Querschnittsfläche senkrecht zu den damit verbundenen Stab-Gelenk-Einheiten seitlich auftretenden Kräften möglichst gut standhalten zu können ist vorgesehen, dass die jeweils mit dem zugeordneten Linearaktor verbindbaren Enden der Stab-Gelenk-Einheiten entlang eines äußeren Umfangs des Instrumentenschafts beabstandet zueinander angeordnet sind. Vorzugsweise weist die Manipulationsvorrichtung eine Befestigungsvorrichtung oder eine Linearführungseinrichtung mit einer kreisförmigen Grundfläche auf, die zudem an die Abmessungen des Instrumentenschafts angepasst sein kann. Die Aufnahme von seitlich, beziehungsweise in radialer Richtung quer zu der Bewegungsrichtung der Linearaktoren auftretender Kräfte gelingt um so besser, je weiter die einzelnen Antriebe, beziehungsweise Linearaktoren räumlich beabstandet zueinander angeordnet sind. Daher sollten die einzelnen Linearaktoren und die zugeordneten Enden der Stab-Gelenk-Einheiten möglichst weit außen entlang des Umfangs des Instrumentenschafts verteilt angeordnet sein. Zudem ist es für die Stabilität der Manipulationsvorrichtung und die mechanische Steifigkeit insbesondere der Werkzeugplattform relativ zu dem Instrumentenschaft vorteilhaft, wenn die verzweigenden Stab-Gelenk-Einheiten einen möglichst großen Abstand zu den durchgehenden Stab-Gelenk-Einheiten aufweisen, was insbesondere die jeweils zugeordneten und mit den Linearaktoren verbindbaren Enden der Stab-Gelenk-Einheiten betrifft.

Um eine einfache Montage der austauschbaren und gegebenenfalls als Einwegartikel ausgestalteten Manipulationsvorrichtung an dem Instrumentenschaft zu ermöglichen und beispielsweise eine falsche zuordnung der einzelnen Enden der Stab-Gelenk-Einheiten zu den Linearaktoren vermeiden zu können ist vorgesehen, dass die jeweils mit dem zugeordneten Linearaktor verbindbaren Enden der Stab-Gelenk-Einheiten unsymmetrisch angeordnet sind.

Vorzugsweise ist vorgesehen, dass die Werkzeugplattform und die Stab-Gelenk-Einheiten aus einem geeigneten Kunststoff hergestellt sind. Das hierzu verwendete Kunststoffmaterial ist vorzugsweise biokompatibel und von ausreichend mechanischer Festigkeit. Um die Herstellung der einzelnen Elemente der Manipulationsvorrichtung zu erleichtern, sollte das verwendete Kunststoffmaterial mit herkömmlichen spritzgusstechnologien verarbeitet werden können. Thermoplaste wie beispielsweise Polypropylen sind für viele denkbare Anwendungen ein geeignetes Kunststoffmaterial.

Gemäß einer besonders vorteilhaften Ausgestaltung des Erfindungsgedankens ist vorgesehen, dass die Manipulationsvorrichtung einstückig ausgeführt ist. Viele verschiedene Gelenkarten und insbesondere Knickgelenke, bzw. Rotationsgelenke, die ein Verschwenken um eine Schwenkachse ermöglichen, lassen sich durch eine geeignete Schwächung bzw. Ausnehmung in einem stabförmigen Bereich eines Kunststoffteils herstellen bzw. bewerkstelligen. Auch aus diesem Grund ist die Verwendung von Gelenken mit einem rotatorischen Freiheitsgrad, wie beispielsweise Knickgelenke oder Schwenkgelenke, besonders vorteilhaft, da diese mit geringem Aufwand hergestellt werden und über einen langen zeitraum hinweg zuverlässig genutzt und angesteuert werden können. Eine einstückig ausgeführte Manipulationsvorrichtung lässt sich kostengünstig herstellen und ist deshalb auch für eine vorgesehene Verwendung als Einmalartikel besonders geeignet.

Die Erfindung betrifft auch ein chirurgisches Instrument mit einem Instrumentenschaft, an dem zwei oder mehrere Manipulationsvorrichtungen gemäß den vorangehenden Ausführungen angeordnet und unabhängig voneinander betätigbar sind. Die zwei oder mehreren Manipulationsvorrichtungen können entlang eines Umfangs des Instrumentenschafts angeordnet sein, während mittig an dem Instrumentenschaft, bzw. in dem Inneren des Instrumentenschaftes eine Beleuchtungseinrichtung und eine Kamera, bzw. eine bildgebende Einrichtung angeordnet werden können.

Nachfolgend werden Ausführungsbeispiele des Erfindungsgedankens näher erläutert, die in der Zeichnung dargestellt sind. Es zeigt:

Fig. 1 eine schematische Darstellung der für eine Manipulationsvorrichtung vorgesehenen intrakorporalen Freiheitsgrade,

Fig. 2 eine schematische Darstellung einer Seitenansicht einer Manipulationsvorrichtung mit zwei durchgehenden Stab-Gelenk-Einheiten und zwei verzweigenden Stab-Gelenk-Einheiten,

Fig. 3 eine andere Seitenansicht der in Fig. 2 dargestellten Manipulationsvorrichtung,

Fig. 4 eine realitätsnahe Seitenansicht der in den Fig. 2 und 3 schematisch dargestellten Manipulationsvorrichtung mit zwei durchgehenden Stab-Gelenk-Einheiten und mit zwei verzweigenden Stab-Gelenk-Einheiten,

Fig. 5 eine schematische Schnittansicht der in Fig. 4 abgebildeten Manipulationsvorrichtung längs der Linie V-V und

Fig. 6 eine schematische Darstellung eines chirurgischen Instruments mit zwei Manipulationsvorrichtungen, die über eine Befestigungsvorrichtung mit einem Instrumentenschaft verbunden sind.

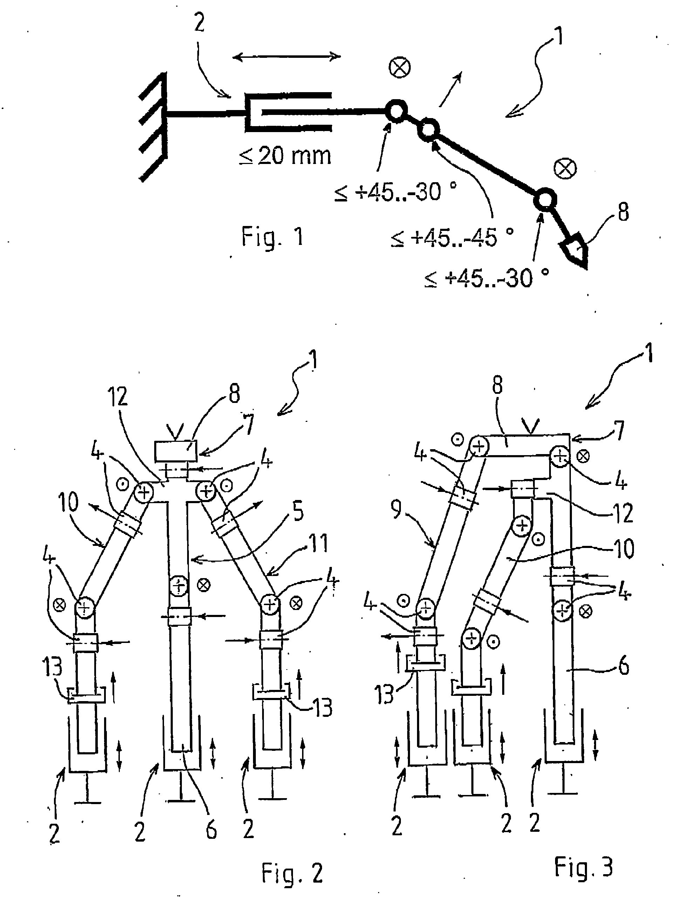

Vorausgegangene eigene Untersuchungen und Überlegungen haben ergeben, dass eine Manipulationsvorrichtung mit den in Fig. 1 schematisch dargestellten intrakorporalen Freiheitsgraden einen vorteilhaften Kompromiss zwischen den von einem Chirurgen gewünschten großen Bewegungsmöglichkeiten einer Manipulationsvorrichtung und der möglichst geringen Komplexität dieser Manipulationsvorrichtung darstellen.

Da die Manipulationsvorrichtung im Wesentlichen von Linearaktoren angetrieben werden soll, beinhaltet der erste Freiheitsgrad in nahe liegender Weise eine lineare Verschiebung in Verlängerung des Instrumentenschafts von beispielsweise bis zu 20 mm. Im Anschluss daran folgen drei eindimensionale rotatorische Freiheitsgrade, die jeweils gegenüber dem vorausgehenden Gelenk um 90° versetzt sind. Derartige rotatorische Freiheitsgrade haben erfahrungsgemäß den höchsten Nutzwert für den Chirurg und erlauben gleichzeitig eine einfache und kostengünstige Ausgestaltung im Rahmen einer monolithischen bzw. einstückigen Anfertigung der Manipulationsvorrichtung. Neben der Linearbewegung in Verlängerung des Instrumentenschafts können demzufolge drei Knickbewegungen ausgeführt werden, die jeweils zu der vorausgehenden Knickbewegung einen Winkel von 90° aufweisen. Die in Fig. 1 jeweils genannten Schwenkbereiche um die einzelnen Rotationsgelenke stellen lediglich zur Veranschaulichung eine beispielhafte, in der Praxis als vorteilhaft empfundene Konfiguration dar.

Für den gezielten Antrieb bzw. die Betätigung von einer Manipulationsvorrichtung 1, wie sie in den Fig. 2 und 3 dargestellt ist, werden vier Linearaktoren 2 benötigt. Alle Linearaktoren 2 sind gestellfest an einem nicht dargestellten Instrumentenschaft befestigt. An dem Instrumentenschaft sind demzufolge mittels einer ebenfalls nicht dargestellten Befestigungsvorrichtung vier Stab-Gelenk-Einheiten 3 über die zugeordneten Linearaktoren 2 befestigt. Die einzelnen Stab-Gelenk-Einheiten 3 weisen jeweils ausschließlich ein-dimensionale Rotationsgelenke 4 auf. Eine durchgehende Stab-Gelenk-Einheit 5 weist neben dem zugeordneten Linearaktor 2 drei eindimensionale Rotationsgelenke 4 auf, deren Schwenkachse jeweils in einem Winkel von 90° relativ zueinander versetzt ist. Diese durchgehende Stab-Gelenk-Einheit 5 wird auch als Hauptkette bezeichnet.

Die durchgehende Stab-Gelenk-Einheit 5 ist an ihrem einen Ende 6 mit dem zugeordneten Linearaktor 2 und damit auch mit dem Instrumentenschaft verbunden und an einem anderen Ende 7 mit einer Werkzeugplattform 8 verbunden. Die einzelnen Rotationsgelenke 4 der Hauptkette entsprechen demzufolge neben der durch den zugeordneten Linearaktor 2 bewirkten linearen Auslenkung den in Fig. 1 schematisch dargestellten Freiheitsgraden der Manipulationsvorrichtung 1.

Eine weitere durchgehende Stab-Gelenk-Einheit 9 ist ebenfalls sowohl mit dem zugeordneten Linearaktor 2 als auch mit der Werkzeugplattform 8 verbunden. Zwei verzweigende Stab-Gelenk-Einheiten 10, 11 sind an einem Verzweigungsabschnitt 12 mit der Hauptkette 5 verbunden und weisen keine direkte Verbindung zu der Werkzeugplattform 8 auf. Die durchgehende Stab-Gelenk-Einheit 9 und die verzweigenden Stab-Gelenk-Einheiten 10, 11 bilden Antriebsketten, mittels derer die Werkzeugplattform 8 nach den Vorgaben des Benutzers innerhalb ihres Arbeitsraums bewegt werden kann.

Die Hauptkette 5 weist drei eindimensionale, um eine Drehachse verschwenkbare Rotationsgelenke 4 auf. Die für eine mechanisch bestimmte kinematische Struktur vorgegebenen Gelenkfreiheitsgrade müssen in geeigneter Weise auf die Hauptkette 5 und die Antriebsketten 9, 10, 11 verteilt werden. Die Anzahl der zu verteilenden Gelenkfreiheitsgrade beträgt ohne Berücksichtigung von redundanten Freiheitsgraden und Zwangsbedingungen

$$ f = F - 6 \times (1 + v - k), $$

wobei f der Gesamtzahl der Gelenkfreiheitsgrade entspricht, F die Anzahl der Freiheitsgrade der kinematischen Struktur bezeichnet, v die Anzahl der Verzweigungen und k die Anzahl der kinematischen Ketten bezeichnet, die den in diesem Zusammenhang beschriebenen stab-Gelenk-Einheiten entsprechen. Für den vorliegenden Fall ergibt sich mit den vorangehend bereits beschriebenen Annahmen F = 4, k = 4 und v = 1 die Gesamtzahl der Gelenkfreiheitsgrade

$$ f = 22. $$

Der schematischen Darstellung in den Fig. 2 und 3 kann entnommen werden, wie bei dem abgebildeten Ausführungsbeispiel die den jeweiligen Stab-Gelenk-Einheiten 5, 9, 10 und 11 zugeordneten Gelenkfreiheitsgrade durch eine entsprechende Anordnung von überwiegend eindimensionalen Rotationsgelenken 4 verteilt bzw. vorgegeben werden. Dabei ist in den Antriebsketten 9, 10 und 11 jeweils ein Gelenk als Torsionsgelenk 13 ausgestaltet, dessen einer rotatorischer Freiheitsgrad eine Verdrehung der beiden benachbarten Stabelemente relativ zueinander um eine Längsachse ermöglicht.

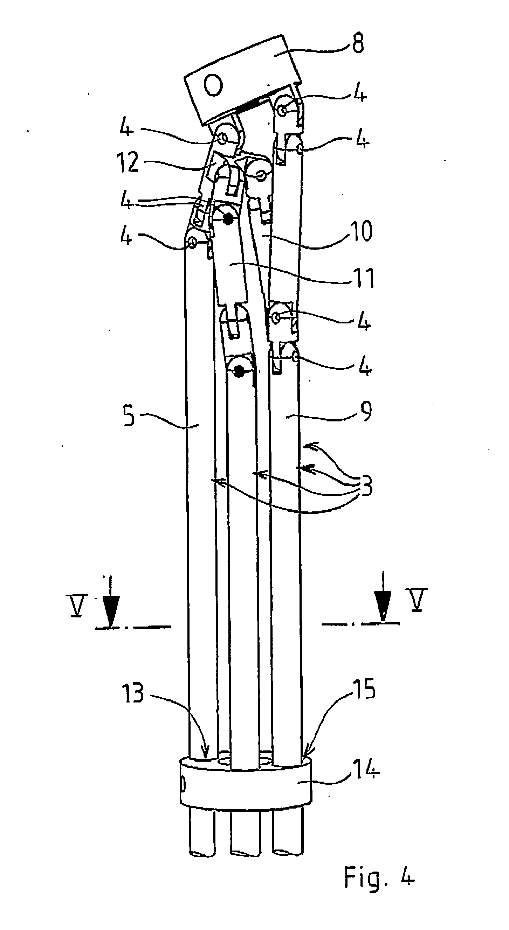

Fig. 4 zeigt eine Seitenansicht eines exemplarischen Prototypen für die Manipulationsvorrichtung 1. Ausgehend von nicht abgebildeten Linearaktoren 2 weist die Manipulationsvorrichtung 1 vier Stab-Gelenk-Einheiten 3 auf. Jede Stab-Gelenk-Einheit 3 weist ihrerseits ausschließlich eindimensionale Rotationsgelenke 4 auf. Die Antriebsketten 9, 10 und 11 weisen dabei jeweils ein Torsionsgelenk 13 auf. Im Hinblick auf eine verbesserte (Seiten-) Stabilität der Manipulationsvorrichtung 1 weist diese eine Führungseinrichtung 14 mit an die Stab-Gelenk-Einheiten 3 angepassten durchgehenden Bohrungen 15 auf. Die Torsionsgelenke 13 sind längsverschiebbar innerhalb der Führungseinrichtung 14 angeordnet.

Sowohl die Hauptkette 5 als auch die weitere durchgehende Stab-Gelenk-Einheit 9 sind jeweils über ein eindimensionales Rotationsgelenk mit der Werkzeugplattform 8 verbunden bzw. daran gelagert. Die beiden verzweigenden Stab-Gelenk-Einheiten 10, 11 sind im Bereich des Verzweigungsabschnitts 12 mit der Hauptkette 5 verbunden.

Obwohl die in Fig. 4 dargestellte Manipulationsvorrichtung 1 aus mehreren gelenkig miteinander verbundenen Stäben und einer ebenfalls gelenkig verbundenen Werkzeugplattform 8 aufgebaut ist, kann eine vergleichbare Manipulationsvorrichtung 1 mit identischer Funktionsweise und vergleichbarem Arbeitsraum auch einstückig ausgeführt werden. Eine aus einem geeigneten Thermoplast kostengünstig hergestellte, einstückige Manipulationsvorrichtung 1 ist besonders vorteilhaft für eine Verwendung als Einwegartikel geeignet, der unmittelbar vor Beginn des minimalinvasiven Eingriffs einer sterilen Verpackung entnommen und mit dem mehrfach verwendbaren Instrumentenschaft verbunden werden kann. Im Anschluss an den chirurgischen Eingriff kann die Manipulationsvorrichtung 1 von dem Instrumentenschaft gelöst und entweder gereinigt und erneut sterilisiert oder aber entsorgt werden.

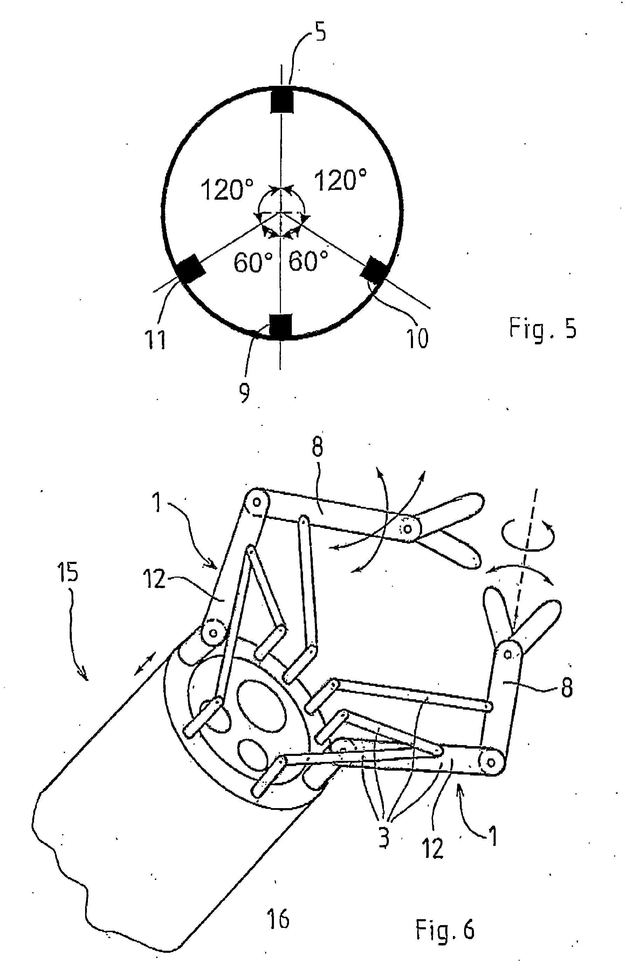

Um eine fehlerfreie Zuordnung der einzelnen Stab-Gelenk-Einheiten 3 zu den jeweils zugeordneten Linearaktoren 2 bzw. eine rasche und unkomplizierte Befestigung der Manipulationsvorrichtung 1 an dem Instrumentenschaft zu erleichtern, sind von den längs eines Umfangs einer im Wesentlichen kreisförmigen Querschnittsfläche des Instrumentenschafts angeordneten Stab-Gelenk-Einheiten 3 die verzweigenden Stab-Gelenk-Einheiten 10, 11 nicht äquidistant zu den jeweils benachbarten Stab-Gelenk-Einheiten 5 und 9 angeordnet. Eine schematische Darstellung der Anordnung der einzelnen Stab-Gelenk-Einheiten 3 an der Befestigungsvorrichtung, bzw. an dem Instrumentenschaft ist in Fig. 5 abgebildet. Die Abweichung von einer vollständig symmetrischen Anordnung der einzelnen Stab-Gelenk-Einheiten 5, 9, 10 und 11 erleichtert die fehlerfreie Zuordnung und Befestigung der Manipulationsvorrichtung 1, ohne dass zusätzliche Zuordnungs- oder Befestigungselemente erforderlich sind.

Fig. 6 zeigt eine schematische Darstellung eines exemplarisch ausgestalteten chirurgischen Instruments 15 mit einem Instrumentenschaft 16, der eine kreisrunde Querschnittsfläche aufweist. Über eine nicht dargestellte Befestigungsvorrichtung sind zwei Manipulationsvorrichtungen 1 lösbar mit dem Instrumentenschaft 16 verbunden. Beide Manipulationsvorrichtungen 1 sind zur Veranschaulichung gleich ausgestaltet und entsprechen hinsichtlich der Funktionalität den in den Fig. 2 bis 4 dargestellten Manipulationsvorrichtungen 1. Natürlich können die Manipulationsvorrichtungen 1 jeweils unterschiedliche und an die jeweiligen Anforderungen angepasste Stab-Gelenk-Einheiten 3 und ebenso unterschiedliche Werkzeuge an der jeweiligen werkzeugplattform 8 aufweisen. Die einzelnen Stab-Gelenk-Einheiten 3 der beiden Manipulationsvorrichtungen 1 sind entlang einer Umfangslinie in einem umlaufenden Randbereich der Querschnittsfläche des Instrumentenschafts 16 mit den ebenfalls nicht dargestellten, im Inneren des Instrumentenschafts 16 festgelegten Linearaktoren antreibbar verbunden.

**Patentansprüche**

1. Manipulationsvorrichtung (1) für ein chirurgisches Instrument mit einer Befestigungseinrichtung zur Befestigung der Manipulationsvorrichtung (1) an einem Instrumentenschaft, an welchem mehrere Linearaktoren (2) festlegbar sind, mit einer Werkzeugplattform (8) zur Aufnahme von mindestens einem chirurgischen Werkzeug und mit mehreren passiven Stab-Gelenk-Einheiten (5, 9, 10, 11), deren eines Ende jeweils mit einem zugeordneten Linearaktor (2) verbindbar ist und mittels derer die Werkzeugplattform (8) mit den Linearaktoren (2) in Wirkverbindung bringbar ist, **dadurch gekennzeichnet, dass** mindestens zwei durchgehende Stab-Gelenk-Einheiten (5, 9) jeweils mit der Werkzeugplattform (8) verbunden sind und dass mindestens eine verzweigende Stab-Gelenk-Einheit (10, 11) an einem Verzweigungsabschnitt (12) beabstandet zu der werkzeugplattform (8) mit einer zugeordneten durchgehenden Stab-Gelenk-Einheit (5) verbunden ist.

2. Manipulationsvorrichtung nach Anspruch 1, **dadurch gekennzeichnet, dass** zwischen dem Verzweigungsabschnitt (12) der mit einer verzweigenden Stab-Gelenk-Einheit (10, 11) verbundenen durchgehenden Stab-Gelenk-Einheit (5) und der Werkzeugplattform (8) mindestens ein Gelenk angeordnet ist.

3. Manipulationsvorrichtung nach Anspruch 1 oder Anspruch 2, **dadurch gekennzeichnet, dass** die Manipulationsvorrichtung (1) zwei verzweigende Stab-Gelenk-Einheiten (10, 11) aufweist.

4. Manipulationsvorrichtung nach Anspruch 3, **dadurch gekennzeichnet, dass** die Manipulationsvorrichtung (1) zwei durchgehende Stab-Gelenk-Einheiten (5, 9) und zwei verzweigende Stab-Gelenk-Einheiten (10, 11) aufweist.

5. Manipulationsvorrichtung nach Anspruch 3 oder 4, **dadurch gekennzeichnet, dass** die beiden verzweigenden Stab-Gelenk-Einheiten (10, 11) mit derselben durchgehenden Stab-Gelenk-Einheit (5) verbunden sind.

6. Manipulationsvorrichtung nach einem der vorangehenden Ansprüche, **dadurch gekennzeichnet, dass** die Stab-Gelenk-Einheiten (5, 9, 10, 11) ausschließlich Rotationsgelenke (4) mit einem rotatorischen Gelenkfreiheitsgrad aufweisen.

7. Manipulationsvorrichtung nach Anspruch 6, **dadurch gekennzeichnet, dass die jeweils benachbarten** Rotationsgelenke (4) relativ zueinander verdrehte Gelenkachsen aufweisen.

8. Manipulationsvorrichtung nach Anspruch 7, **dadurch gekennzeichnet, dass** die Gelenkachsen mehrerer benachbarter Rotationsgelenke (4) innerhalb einer Stab-Gelenk-Einheit (5, 9, 10, 11) einen Winkel von im Wesentlichen 90° relativ zueinander aufweisen.

9. Manipulationsvorrichtung nach einem der vorangehenden Ansprüche, **dadurch gekennzeichnet, dass** jeweils mit dem zugeordneten Linearaktor (2) verbindbare Enden der Stab-Gelenk-Einheiten (5, 9, 10, 11) entlang eines äußeren Umfangs der Befestigungseinrichtung beabstandet zueinander angeordnet sind.

10. Manipulationsvorrichtung nach Anspruch 9, **dadurch gekennzeichnet, dass** die jeweils mit dem zugeordneten Linearaktor (2) verbindbaren Enden (6) der Stab-Gelenk-Einheiten (5, 9, 10, 11) unsymmetrisch angeordnet sind.

11. Manipulationsvorrichtung nach einem der vorangehenden Ansprüche, **dadurch gekennzeichnet, dass** die Werkzeugplattform (8) und die Stab-Gelenk-Einheiten (5, 9, 10, 11) aus einem biokompatiblen Kunststoffmaterial hergestellt sind.

12. Manipulationsvorrichtung nach einem der vorangehenden Ansprüche, **dadurch gekennzeichnet, dass** jede Stab-Gelenk-Einheit (5, 9, 10, 11) einstückig ausgestaltet ist.

13. Manipulationsvorrichtung nach einem der vorangehenden Ansprüche, **dadurch gekennzeichnet, dass** die Manipulationsvorrichtung (1) einstückig ausgeführt ist.

14. Manipulationsvorrichtung nach einem der vorangehenden Ansprüche, **dadurch gekennzeichnet, dass** die Befestigungsvorrichtung (1) eine kreisförmige Grundfläche aufweist, die an die Abmessungen des Instrumentenschafts angepasst ist.

15. Chirurgisches Instrument (15) mit einem Instrumentenschaft (16) und mit einer mit dem Instrumentenschaft (16) verbindbaren Befestigungsvorrichtung, an der zwei oder mehrere Manipulationsvorrichtungen (1) gemäß einem der vorangehenden Ansprüche 1 bis 14 angeordnet und unabhängig voneinander betätigbar sind.

**16.** Chirurgisches Instrument (15) nach Anspruch 15, **dadurch gekennzeichnet, dass** die zwei oder mehreren Manipulationsvorrichtungen (1) entlang eines Umfangs des Instrumentenschafts (16) mit einer kreisförmigen Querschnittsfläche angeordnet sind.

**17.** Chirurgisches Instrument (15) nach Anspruch 15 oder Anspruch 16, **dadurch gekennzeichnet, dass** die zwei oder mehreren Manipulationsvorrichtungen (1) lösbar mit dem Instrumentenschaft (16) verbindbar sind.

Fig. 1

≤ 20 mm

≤ +45..-30 °

≤ +45..-45 °

≤ +45..-30 °

Fig. 2

Fig. 3

Fig. 4

5

120° 120°

60° 60°

11 10

9

Fig. 5

8

1

15

12

8

3 12 1

16

Fig. 6

**IN DER BESCHREIBUNG AUFGEFÜHRTE DOKUMENTE**

**In der Beschreibung aufgeführte Patentdokumente**

- EP 1224918 A
- US 2005096694 A
- US 6309403 B
- US 2001018591 A